# EUROPÄISCHE PATENTSCHRIFT

(11) **EP 0 497 158 B1**
(45) Veröffentlichungstag und Bekanntmachung des Hinweises auf die Patenterteilung: **13.03.1996**
(21) Anmeldenummer: 92100701.9
(22) Anmeldetag: 17.01.1992
(51) Int. Cl.: A61K 31/60, A61K 9/00

(54) **Pharmazeutischer Kaugummi mit Acetylsalicylsäure**
Pharmaceutical chewing-gum containing acetylsalicylic acid
Gomme à mâcher pharmaceutique contenant de l'acide acétylsalicylique

(30) Priorität: 30.01.1991 DE 4102629
(43) Veröffentlichungstag der Anmeldung: 05.08.1992
(73) Patentinhaber: BAYER AG, D-51368 Leverkusen (DE)
(72) Erfinder: Häusler, Franz, Dr., W-5060 Bergisch Gladbach (DE); Maasz, Joachim, Dr., W-5653 Leichlingen 1 (DE); Valéri, Thomas, Dr., W-4047 Dormagen 1 (DE)

(56) Entgegenhaltungen:
- EP-A- 0 151 344
- EP-A- 0 236 271
- WO-A-88/06449
- BE-A- 893 729
- US-A- 4 937 076

## Beschreibung

Die Erfindung betrifft stabile, pharmazeutisch nutzbare Kaugummiformulierungen, die Acetylsalicylsäure (ASS) als Wirkstoff enthalten, und ein Verfahren zu deren Herstellung.

Acetylsalicylsäure ist ein seit langem bekannter Arzneistoff mit fiebersenkenden, entzündungshemmenden und analgetischen Eigenschaften. Über längere Zeiträume und in hohen Dosen wird Acetylsalicylsäure zur Behandlung rheumatischer Erkrankungen eingesetzt. Acetylsalicylsäure wirkt hemmend auf die Thrombozytenaggregation, wobei vermutlich die Acetylierung der Thrombozyten-Cyclooxygenase eine wichtige Rolle spielt. Diese Eigenschaft der Substanz wird bei ihrem Einsatz zur Prophylaxe und Behandlung von Thrombosen und im Bereich von anderen Herz-Kreislauf-Erkrankungen genutzt. In dieser Indikation sind relativ geringe Dosen wirksam.

Acetylsalicylsäure wird relativ langsam resorbiert. Bei oraler Applikation wird das Blutspiegelmaximum erst nach etwa 2 Stunden erreicht. Dabei erfährt die Substanz eine metabolische Veränderung, in deren Verlauf es zur Abspaltung von Essigsäure kommt. Diese Reaktion erfolgt im ersten Schritt durch die Esterasen der Mucosa und im weiteren durch die Esterasen in Leber, Plasma und Erythrozyten. Die langsame Resorption ist wegen der damit einhergehenden hohen Metabolisierungsrate besonders in der Indikation Thrombozytenaggregationshemmung unerwünscht, da hier das unveränderte Acetylsalicylsäuremolekül die Wirkform darstellt.

Ähnlich wie andere organische Säuren wirkt Acetylsalicylsäure lokal reizend und gewebeschädigend. Durch Schädigung der Magenschleimhaut kommt es bei der Anwendung nicht selten zu Mikroblutungen. Zu bedrohlichen Blutungen kann es beim Vorliegen von Ulcerea im Magen-Darmbereich kommen, Dabei wächst die Gefahr einer Magenschleimhautschädigung mit der verabreichten Dosis.

Aus den obengenannten Gründen ist es sinnvoll, nach pharmazeutischen Darreichungsformen für Acetylsalicylsäure zu suchen, die einerseits eine schnelle Resorption gewährleisten und andererseits eine guten Magenverträglichkeit besitzen.

Eine Möglichkeit, die genannten Probleme zu lösen, ist die Verwendung von acetylsalicylsäurehaltigen Brausezubereitungen. Sie haben jedoch folgende Nachteile:
- Zur Einnahme benötigt man ein Glas sauberes Wasser und mindestens 2 bis 3 Minuten Zeit. Sie sind deshalb z.B. nicht für unterwegs geeignet.
- Sie haben normalerweise einen hohen Natriumgehalt.
- Ihre Herstellung und Verpackung in feuchtigkeitsdichte Packmittel muß bei besonders niedriger Luftfeuchtigkeit erfolgen und ist deshalb aufwendig und teuer.
- Brausezubereitungen sind hochpreisige Produkte.

Kaubare Massen (Kaugummi, chewing gum, bubble gum, stick gum) wurden bereits im letzten Jahrhundert beschrieben, ebenfalls sehr lange bekannt sind Kaugummis und Kautabletten mit medizinischer Anwendung. Die erste acetylsalicylsäurehaltige Kaugummiformulierung wurde 1924 in den USA auf den Markt gebracht.

Auch in der Literatur sind Kaugummiformulierungen, die Acetylsalicylsäure enthalten erwähnt. Beispielsweise beschreibt US 2 465 233 einen Kaugummi zur Behandlung von Kinetosen, der eine Kombination von Scopolaminhydrobromid und Acetylsalicylsäure enthält. EP 0 151 344 und EP 0 236 271 beschreiben Kaugummiformulierungen, die pharmazeutische Wirkstoffe, ASS ist genannt, enthalten können. In EP 0 253 040 wird ein Verfahren zur Herstellung eines Kaugummi-Bonbons dargestellt, wobei Arzneistoffe vom Typ der Acetylsalicylsäure als mögliche Inhaltsstoffe bezeichnet werden.

Alle genannten Beispiele haben den gemeinsamen gravierenden Nachteil, daß beim Kauen der Zubereitung im Mund eine Lösung mit niedrigem pH-Wert entsteht. Diese Lösung führt zur Reizung der Mundschleimhaut und zur Schädigung des Zahnschmelzes, insbesondere der letzte Punkt wird heute unter dem Gesichtspunkt der Kariesvorsorge sehr kritisch gesehen.

Eine Lösung für dieses Problem bietet FR 87/02939 an, indem hier das Lysinsalz der Acetylsalicylsäure als Wirkstoff in einer buccalen Arzneiform eingesetzt wird.

Auf die dort beschriebene Weise lassen sich jedoch keine Arzneiformen herstellen, die eine hinreichende chemische Stabilität aufweisen. Die Hydrolysestabilität von Acetylsalicylsäure nimmt mit steigendem pH-Wert ab. Das Stabilitätsoptimum liegt im pH-Bereich 2 bis 3 (vgl. DAB 9, Seite 769 Kommentar). Dort wird auch ausdrücklich darauf hingewiesen, daß ASS unverträglich ist mit alkalisch reagierenden Stoffen. Das bedeutet, alle Salze, einschließlich des Lysinsalzes, haben eine geringere Stabilität als die freie Säure. Da bereits die Herstellung von stabilen Zubereitungen der freien Säure mit großen Schwierigkeiten verbunden ist, ist beim Einsatz von Salzen der Acetylsalicylsäure um so weniger mit einem Erfolg zu rechnen.

Auch in WO-A-8806449 wird vorgeschlagen, die Verträglichkeit durch Verwendung von Acetylsalicylsäure-Lysinat zu verbessern und durch Glycineinsatz eine Erhöhung der Stabilität gegen Hydrolyse zu erreichen. Eine räumliche Trennung der Komponenten wird jedoch nicht beschrieben.

In US-P-4937076 wird eine Kautablette beschrieben, welche Acetylsalicylsäure und einen basischen Puffer enthält, wobei diese Komponenten durch Einlagerung in fettähnliches Material räumlich voneinander getrennt sind und erst nach Verdauung der Fettumhüllung in Gastronintestinal-Trakt miteinander reagieren können.

Wider Erwarten wurde nun gefunden, daß es durch die erfindungsgemäße Zusammensetzung und den Einsatz eines speziellen Herstellungsverfahren möglich ist, stabile, pharmazeutisch nutzbare, ASS-haltige Kaugummiformulierungen herzustellen, die alle oben genannten Nachteile beseitigen und die sich zur Verabreichung aller gebräuchlichen Dosen von ASS eignen. Erstaunlicherweise konnten durch die erfindungsgemäße Formulierung die Vorteile der bereits bekannten buccalen Arzneiformen, nämlich die gute Schleimhaut- und Mucosaverträglichkeit der ein ASS enthaltenden Kauformulierung einerseits und die gute Stabilität der die freie Säure enthaltenden Arzneiformen andererseits, miteinander kombiniert werden, ohne die jeweiligen Nachteile in Kauf nehmen zu müssen.

Die Erfindung betrifft eine stabile, schleimhautverträgliche Kaugummi-Formulierung, die Acetylsalicylsäure als eine Komponente und eine zur Salzbildung geeignete basische Substanz als zweite Komponente in räumlich getrennter Form enthält, wobei die beiden Komponenten während des Kauvorganges aus der Kaugummimasse herauslösbar sind und gutlösliche Salze der Acetylsalicylsäure bilden und die Kaugummibase maximal 2 Gew.-% Wasser enthält, dadurch gekennzeichnet, daß die räumliche Trennung zwischen ASS und der basischen Puffersubstanz durch folgende Maßnahmen gewährleistet ist:
a) die beiden Komponenten sind jeweils unabhängig voneinander in einem adäquaten Teil der Kaugummimasse eingearbeitet und diese "Vorbatche" werden vor der Ausformung gemischt oder
b) die beiden Komponenten sind jeweils unabhängig voneinander in verschiedene Kaugummimassen eingearbeitet, die untereinander teilverträglich sind, so daß bei der gemeinsamen Kompoundierung beider Grundmassen ein zweiphasiges Systeme entsteht.

Während der Lagerung der Zubereitung liegt die ASS als freie Säure vor und hat deshalb eine entsprechend hohe chemische Stabilität, während des Kauvorganges entsteht das Salz der ASS bzw. die Lösung dieses Salzes, welche eine wesentlich verbesserte Schleimhautverträglichkeit als die freie Säure besitzt und den Zahnschmelz weniger angreift.

Die erfindungsgemäße Kaugummiformulierung hat vorzugsweise folgende Zusammensetzung:

| | |
|---|---|
| Acetylsalicylsäure | 2 - 30 Gew.-Teile entsprechend 30-1500 mg |
| Basische Puffersubstanzen | entsprechend 0,1-17 mEq* Pufferkapazität |
| Kaugummibase | 15 - 50 Gew.-Teile |
| Weichmacher | 0 - 30 Gew.-Teile |
| Zucker und/oder | 0 - 55 Gew.-Teile |
| Zuckeraustauschstoffe Süßstoff | 0 - 2 Gew.-Teile |
| Aromastoffe | 0 - 5 Gew.-Teile |
| Füllstoffe | 0 - 30 Gew.-Teile |
| ggf. weitere Komponenten wie Wachse, Emulgatoren, Stabilisatoren | 0 - 20 Gew.-Teile |
| ggf. wasserlösliches Polymer | 0 - 30 Gew.-Teile |

| | |
|---|---|
| * entsprechend USP XXII | |

Kaugummibasen bestehen in der Regel aus zwei Hauptkomponenten, die zum Erzielen der gewünschten Kaugummieigenschaften notwendig sind. Eine Elastomerkomponente A stellt den volumenbildenden, wasserunlöslichen Anteil dar, eine harzartige, ebenfalls wasserunlösliche Komponente B ist für die ständige Kaubarkeit des Materials verantwortlich. Sowohl die Elastomerkomponente A als auch der harzartige Zusatz B können natürlichen oder synthetischen Ursprungs sein. Ebenso ist eine Kombination von natürlichem und synthetischem Material möglich.

Als Elastomerkomponenten A kommen alle dem Fachmann bekannten Elastomere, die physiologisch verträglich sind, in Betracht. Diese können z.B. sein: Naturgummi wie Chicle, Polyvinylacetate, Isobutylen-Isoprencopolymere, Styrol-Butadiencopolymere, Polyisobutylen, Guttapercha, Crown Gummi, Polyisopren, Polyethylen, natürliche Polyterpene sowie Mischungen aus diesen.

Üblicherweise verwendete Harzkomponenten B sind z.B. Arkon P, Polyvinylester geeigneten Molekulargewichts (z.B. Polyvinylacetat MG 20.000), Copolymere von Vinylestern und Vinylethern, Polyethylen-Vinylacetatcopolymere und Naturharze wie z.B. Dammar und Gajak.

Gemäß der vorliegenden Erfindung können für die Kaugummizusammensetzung als Grundlagen auch kommerziell verfügbare Kaugummibasen verwendet werden. Als basische Puffer-Komponente können Erdalkalicarbonate, vorzugsweise Calciumcarbonat, eingesetzt werden; es kann aber ebenso Calciumhydroxyd, Magnesiumhydroxid, Magnesiumcarbonat leicht, Magnesiumcarbonat schwer oder Magnesiumoxid verwendet werden. Weitere mögliche basische Komponenten sind z.B. Tris-(hydroxymethyl)-aminomethan, Alkali- oder Erdalkaliphosphate und basische Aminosäuren. Die Menge der basischen Komponente wird erfindungsgemäß so gewählt, daß zusammen mit der eingesetzten Menge Acetylsalicylsäure eine Pufferkapazität zwischen 5 und 15 mEq resultiert.

Gegebenenfalls können in die Kaugummibase weitere dem Fachmann bekannte Bestandteile zum Weichmachen und Texturieren, z.B. Fette und Wachse, Emulgieren, z.B. Lecithin, Füllen z.B. Talkum, Aromatisieren und/oder zur Einstellung anderer erforderlicher Eigenschaften eingearbeitet werden. Die Kaugummis können zuckerfrei oder zuckerhaltig sein. Zur Süßung geeignete Verbindungen sind Zucker und Zuckeraustauschstoffe wie Mono- und Disaccaride, Hydrolysate von hochmolekularen Kohlehydraten und Zuckeralkohole. Diese Stoffe können auch ganz oder teilweise durch Süßstoffe wie z.B. Saccharin, Cyclamat oder Aspartam ersetzt werden.

Die Kaugummibase, bestehend aus den Komponenten A und B einschließlich der genannten Zuschlagstoffe, wird im weiteren als Kaugummimasse bezeichnet.

Die üblicherweise zwingend erforderlichen Bestandteile einer Kaugummiformulierung sind die in Wasser unlösliche, inerte Kaugummibase und der wasserlösliche Anteil, der beim Kauen durch den Speichel allmählich aus der Kaugummimatrix gelöst wird. Die Bildung einer (Salz-)-Lösung von ASS im Speichel aus der erfindungsgemäßen Zubereitung im Mund ist aus folgenden Gründen erwünscht:
- Acetylsalicylsäure gelangt so nur in gelöster Form in den Magen, so daß die Ausbildung von Bereichen hoher Wirkstoffkonzentrationen im Magen vermieden wird. Dadurch wird eine optimale Magenverträglichkeit erreicht, die im Bereich einer Brausetablette liegt.
- Aus der Speichellösung können abhängig von der Verweilzeit im Mund schon bedeutende Mengen der Substanz über die Mundschleimhaut resorbiert werden. Auf diese Weise gelangt Acetylsalicylsäure schnell und unmetabolisiert in den Kreislauf und kann dort schnell und effektiv ihre Wirkung entfalten.

Das bereits genannte Problem, daß die im Mund entstehende Wirkstofflösung zu einer Schädigung der Mundschleimhaut und insbesondere der Zähne führen kann, wird erfindungsgemäß durch den Zusatz einer basischen Komponente gelöst, die den Kaugummi zu einer gepufferten Zubereitung macht. Rasterelektronenmikroskopische Untersuchungen an extrahierten, menschlichen Zähnen zeigen, daß die durch Lösungen von Acetylsalicylsäure verursachten Schäden am Zahnschmelz durch eine geeignete Pufferung drastisch reduziert werden und in den Bereich einer Placebolösung gebracht werden können. Auch die für einige bisher bekannte Kautabletten beschriebene Gefahr, daß z.B. über Nacht in der Backentasche verbliebene Tablettenstückchen schwere Entzündungen der Mundschleimhaut verursachen können, besteht bei der neuen Kaugummiformulierung nicht, da Acetylsalicylsäure nur in gelöster und damit gut beweglicher Form aus der Kaugummimatrix austritt und gleichzeitig gepuffert ist. Lokal toxische Konzentrationen sind daher bei der erfindungsgemäßen Form nicht zu befürchten.

Um die bekannte hydrolytische Zersetzung der Acetylsalicylsäure und insbesondere ihrer Salze, die durch Wärme, Feuchtigkeit und alkalisch reagierende Stoffe wesentlich beschleunigt wird, zu verhindern, werden bei der erfindungsgemäßen Herstellung der Zubereitung folgende Maßnahmen einzeln oder kombiniert angewendet:
1. der Wassergehalt des Produktes wird möglichst niedrig gehalten,
2. die Wärmebelastung wird während der Herstellung gering gehalten und
3. die Acetylsalicylsäure wird räumlich von der basischen Komponente getrennt.

### Zu 1.:

Für die erfindungsgemäße Kaugummiformulierung wird eine Kaugummibase mit einem Wassergehalt von maximal 2 %, vorzugsweise bis zu 1 %, insbesondere bis zu 0,3 % (Gewichtsprozent) verwendet. Um den gewünschten niedrigen Wassergehalt zu erreichen, sollen insbesondere die Kaugummibasen, die Weichmacher und die Süßungsmittel wasserarm und wenig hygroskopisch sein.

### Zu 2.:

Häufig werden bei der Herstellung von Kaugummi Temperaturen von mehr als 90°C angewendet. Demgegenüber wird bei der erfindungsgemäßen Herstellung des beschriebenen Kaugummis eine Maximaltemperatur von 85°C nicht überschritten. Die ideale Prozeßtemperatur für die Herstellung liegt bei 40°C, der erfindungsgemäße bevorzugte Temperaturbereich liegt zwischen 20 und 85°C, insbesondere zwischen 30 und 60°C.

### Zu 3.:

a) Der direkte Kontakt zwischen der Wirkstoff-Komponente ASS und der basischen Komponente in der Formulierung wird dadurch verhindert, daß die beiden Komponenten unabhängig voneinander einzeln in eine relativ große Menge Kaugummimasse eingearbeitet werden, so daß der größte Teil der Säure-Partikel einzeln in der Matrix eingebettet ist und durch die Kaugummibase oder die anderen Zuschlagstoffe von seinen benachbarten basischen Partikeln getrennt ist und vice versa. Durch den erfindungsgemäß geringen Wassergehalt der Grundlage ist mit Lösungsvorgängen und einer Diffusion über die flüssige Phase nicht zu rechnen.
b) Eine weitere Möglichkeit der räumlichen Trennung beider Komponenten besteht darin, zunächst die basische Komponente in die eine Hälfte der Kaugummimasseeinzuarbeiten. Anschließend erfolgt die ASS-Einarbeitung in die zweite Hälfte der entsprechenden Kaugummimasse. Ein Kontakt zwischen beiden Verbindungen wird bei diesem Herstellverfahren weitgehend vermieden, da lediglich bei der anschließenden gemeinsamen Abmischung und Formgebung der beiden "Vorbatche" eine reduzierte Kontaktmöglichkeit besteht. Auch in diesen Mischungen liegt der größte Teil der Pulverpartikel isoliert eingebettet in der Kaumasse vor. Somit stellt dieses mit a) vergleichbare Verfahren aufgrund der geänderten Mischweise eine weitere Verbesserung dar.
c) Eine deutlich verbesserte räumliche Trennung kann auch dadurch erreicht werden, daß die unter c) beschriebenen "Vorbatche" in einem abschließenden Formgebungsvorgang zusammengebracht werden, ohne daß hierbei eine direkte Vermischung der Massen erfolgt. Hierfür geeignete Verfahren sind u.a. die Coextrusion über Mehrschichtwerkzeuge (Adapter- und bes. Düsen-Coextrusion zu Mehrschichtfolien/Platten) sowie z.B. Kalanderverfahren zur Herstellung von Mehrschichtsystemen. Zum Einsatz können hierzu alle in der Kautschuk- bzw. Lebensmittelindustrie geeigneten Geräte kommen.
   Der besondere Vorteil eines solchen Herstellverfahrens liegt darin, daß die unter a) beschriebenen Basismassen, von denen die eine die basische Komponente und die andere das ASS enthält, lediglich über die "Grenzflächen" der Coextrudate bzw. Folien in Kontakt treten. Dieses Verfahren bietet sich daher besonders für Formulierungen mit hoher Wirkstoffkonzentration an, da ein Vermischen der Basismassen während der Herstellung ausgeschlossen ist. Somit liegt jedes einzelne Partikel isoliert in der Matrix vor, was eine hohe Lagerstabilität gewährleistet.
d) Eine weitere Möglichkeit der räumlichen Trennung beider Einzelkomponenten besteht darin, den unter c) beschriebenen Vorgang des getrennten Einmischens beider Komponenten in eine Basiskaumasse derart zu modifizieren, daß zunächst die eine Komponente in der üblichen Weise in die Kaugummimasse eingearbeitet wird. Die zweite Komponente wird danach in eine mit der ersten Kaugummimasse nur teilverträgliche, physiologisch unbedenkliche Polymermasse eingearbeitet. Anschließende gemeinsame Kompoundierung beider Grundmassen auf üblichen Mischaggregaten wie z.B. Kneter, Walze oder Extruder führt aufgrund der vorhandenen Teilverträglichkeiten beider Polymersysteme zu allgemein bekannten morphologischen Strukturen. In diesen liegt z.B. eine Komponente dispers verteilt in der anderen vor. Eine solche Struktur ist im Prinzip mit der unter d) beschriebenen verwandt, da auch hier lediglich ein Kontakt über "Grenzflächen" (Matrix und disperse Teile) möglich ist. Diese Strukturen bieten jedoch den Vorteil, daß hier bereits eine enge räumliche Nähe beider Komponenten über den gesamten homogen gemischten Blend besteht, ohne daß es zu einem Kontakt beider Komponenten kommen könnte. Somit kann die gewünschte Salzbildung bei einem anschließenden Kauvorgang sehr viel schneller stattfinden.

Die Kaugummimassen werden erhalten, indem man die Kaugummibase, gegebenenfalls die weiteren Zuschlagstoffe, die Acetylsalicylsäure und die (gegebenenfalls umhüllte) basische Komponente in einem Mischaggregat in Kontakt bringt. Besonders bevorzugt werden Feststoffpartikel mit einer Korngröße von weniger als 50 µm. Höhere Korngrößen lassen sich zwar problemlos einarbeiten, es ist aber mit einem veränderten Kaugefühl zu rechnen.

Die Herstellung der erfindungsgemäßen Kaugummimassen kann nach verschiedenen Verfahren erfolgen. Es kann diskontinuierlich oder kontinuierlich gearbeitet werden. Übliche Verfahren sind z.B. die Herstellung auf Mischwalzen, Knetern und Extrudern. Allgemein geeignet zur Herstellung der erfindungsgemäßen Kaugummimassen sind alle üblichen Geräte und Methoden zur Herstellung von Kaugummi. Die Verarbeitung der Kaugummimassen zu Streifen, Tabletten oder Kugeln und deren Verpackung erfolgt ebenfalls nach üblichen Methoden und ist auf jeder bekannten, zur Kaugummiformung und Verpackung geeigneten Maschine möglich.

Die erfindungsgemäßen Kaugummimassen werden bevorzugt hergestellt, indem man die Kaugummibase auf einer beheizbaren Walze in einen Temperaturbereich von 20 -85°C für einige Minuten mit einer Walzengeschwindigkeit von ca. 10 - 40 Upm walzt, anschließend Acetylsalicylsäure, Geschmackstoffe, Weichmacher und gegebenenfalls sonstige Hilfsstoffe zugibt und gegebenenfalls anschließend basische Puffersubstanz direkt zugibt oder in einem separaten Verfahrensschritt die basische Puffersubstanz in einen Anteil Kaugummibase auf einer entsprechend beheizten Walze einarbeitet, wobei die Kompoundierzeit jeweils zwischen 3 und 15 Minuten liegt, die Kaugummimasse anschließend von der Walze abzieht und nach Abkühlen auf Raumtemperatur in üblicher Weise zu fertigen Kaugummiformulierungen weiterverarbeitet.

Die Walzentemperatur soll zu keinem Zeitpunkt 85°C, vorzugsweise 60°C, überschreiten. Falls erforderlich, wird bei Auftreten von zu starker Friktionswärme Walzentemperatur durch Kühlung herabgesetzt.

Die so hergestellten erfindungsgemäßen Kaugummiformulierungen bieten den Vorteil einer schnellen Resorption der bereits im Mund gelöst vorliegenden Acetylsalicylsäure, den Vorteil einer guten Verträglichkeit im Mund und im Magen durch Salzbildung und Pufferung der entstehenden Lösung während des Kauvorganges, den Vorteil einer hohen Bioverfügbarkeit durch geringe Metabolisierung bei buccaler Absorption und den Vorteil einer hohen Lagerstabilität, da die Acetylsalicylsäure während der Lagerung als freie Säure vorliegt. Die Nachteile der bisher bekannten ASS-haltigen Arzneiformen, die jeweils nur einen oder maximal zwei der genannten Vorteile bieten, werden durch die erfindungsgemäße Formulierung vermieden.

### Ausführungsbeispiele

### Beispiel 1 (Variante 3a)

| | |
|---|---|
| Cafosa Gum Base TAB-3-T | 32 g |
| Zucker | 44 g |
| Cafosa Plasticiser 1001-01 | 3 g |
| Optamint Pfefferminz (H&R) | 4,2 g |
| Citronensäure | 0,8 g |
| ASS | 10 g |
| Calciumcarbonat | 6 g |

32 g einer Kaugummibase (Cafosa Gum Base TAB-3-T) werden auf eine 50°C warme Walze aufgebracht und für 3 Min. gewalzt. Anschließend erfolgt die Zugabe von 44 g Zucker. Nach ca. 3 Min. erhält man eine homogene Masse, in die die übrigen Komponenten einzeln nacheinander eingearbeitet werden. Zunächst werden der Pfefferminzgeschmacksstoff (4,2 g) und die Citronensäure (0,8 g) hinzugegeben. Danach erfolgt die Einarbeitung von 3 g Cafosa Pasticiser 1001-01, 6 g basischer Puffersubstanz (Calciumcarbonat) und 10 g ASS. Nach einer Walzenzeit von insgesamt 10,5 Min. wird die Kaugummimasse von der Walze abgezogen. Nach dem Abkühlen auf Raumtemperatur kann das fertige Material zu jeder gewünschten Form weiterverarbeitet werden.

### Beispiel 2 (Variante 3b)

| | |
|---|---|
| Cafosa Gum Base Dorada Plus-T | 30 g |
| Zucker | 15 g |
| Sorbitpulver | 29 g |
| Ascorbinsäure | 1 g |
| ASS | 15 g |
| Calciumcarbonat | 10 g |

Zur Herstellung dieser Kaugummimasse werden 30 g Cafosa Gum Base Dorada Plus-T auf einer Laborwalze bei 45°C für 3 Min. gewalzt. Danach erfolgt die Zugabe von 15 g Zucker, 29 g Sorbitpulver und 1 g Ascorbinsäure. Es wird weitere 4 Min. bis zur Erhaltung eines homogenen Materials gewalzt. Anschließend wird die Kaugummimasse in zwei Hälften geteilt (Vorbatch 1 und 2). In die eine Hälfte (Vorbatch 1) werden während einer Walzzeit von 2,5 Min. 15 g ASS eingearbeitet, in die andere Hälfte (Vorbatch 2) 10 g Calciumcarbonat als basische Puffersubstanz. Hierdurch wird erreicht, daß in diesen Mischungen der größte Teil der ASS- und Basen-Partikel isoliert eingebettet in der Kaugummimasse vorliegt. An die Herstellung der Vorbatche 1 und 2 schließt sich ein sehr kurzer gemeinsamer Mischvorgang und die nachfolgende Formgebung an.

### Beispiel 3 (Variante 3c)

| | |
|---|---|
| Cafosa Gum Base TAB-3-T | 28 g |
| Sorbitpulver | 39,5 g |
| Na-Cyclamat | 0,5 g |
| ASS | 20 g |
| Calciumcarbonat | 12 g |

Gemäß Beipiel 2 werden zunächst zwei Vorbatche hergestellt, von denen der eine der Rezeptur entsprechende Gesamtmenge ASS und der andere Vorbatch die der Rezeptur entsprechende Gesamtmenge Calciumcarbonat enthält. Die beiden Vorbatche werden einem Aggregat zur Herstellung von Mehrschichtensystemen zugeführt und derart zusammengebracht, daß hierbei keine direkt Vermischung der Massen erfolgt. Hierfür werden die Vorbatche zwei getrennten Einfüllvorrichtungen eines Coextruders zugeführt und anschließend bei einer Düsentemperatur von max. 85°C zu Strängen coextrudiert.

### Beispiel 4

| | |
|---|---|
| Kaugummibase 1 | 13 g |
| Kaugummibase 2 | 14 g |
| Cafosa Plasticiser 1001-01 | 3 g |
| Zucker | 15 g |
| Sorbitpulver | 29 g |
| Ascorbinsäure | 1 g |
| ASS | 15 g |
| Calciumcarbonat | 10 g |

Zur Herstellung dieser Kaugummimasse werden zwei verschiedene Kaugummibasen eingesetzt, die miteinander lediglich teilverträglich sind. Es sind hierzu prinzipiell alle zur Kaugummi-Basenherstellung geeigneten Polymere einsetzbar (vgl. S. 7), die über eine gegenseitige Teilverträglichkeit verfügen.

Zur Herstellung dieser erfindungsgemäßen Kaugummimasse wurden 13 g einer Kaugummibase 1, die Naturkautschuk enthält, bei 85°C auf einer Laborwalze für 3 Min. gewalzt. Danach erfolgt die Zugabe von 7,5 g Zucker, 14,5 g Sorbitpulver, 1,5 g Cafosa Plasticiser, 15 g ASS und 1 g Ascorbinsäure. Es wird weitere 4 Min. bis zum Erhalt enes homogenen Materials gewalzt (Vorbatch 1). Anschließend wird nach derselben Vorgehensweise ein weiterer Vorbatch hergestellt, der aus 14 g der Styrobutadiencopolymer-haltigen Kaugummibase 2, 7,5 Zucker, 14,5 Sorbitpulver, 1,5 g Cafosa Plasticiser und 10 g Calciumcarbonat besteht (Vorbatch 2). Abschließende gemeinsame Kompoundierung beider Vorbatche in einem Mischkneter führt aufgrund vorhandener Teilverträglichkeiten beider Kaugummibasen zu allgemein bekannten morphologischen Strukturen, in denen die eine Kaugummibase dispers verteilt in der anderen vorliegt.

## Patentansprüche (Patentansprüche für folgende(n) Vertragsstaat(en): AT, BE, CH, DE, DK, FR, GB, IT, LI, LU, NL, SE)

1. Stabile, schleimhautverträgliche Kaugummiformulierung, die Acetylsalicylsäure als eine Komponente und eine zur Salzbildung geeignete basische Substanz als zweite Komponente in räumlich getrennter Form enthält, wobei die beiden Komponenten während des Kauvorganges aus der Kaugummimasse herauslösbar sind und gutlösliche Salze der Acetylsalicylsäure bilden und die Kaugummimasse maximal 2 Gew.-% Wasser enthält, dadurch gekennzeichnet, daß die räumliche Trennung zwischen ASS und der basischen Puffersubstanz durch folgende Maßnahmen gewährleistet ist:
a) die beiden Komponenten sind jeweils unabhängig voneinander in einem adäquaten Teil der Kaugummimasse eingearbeitet und diese "Vorbatche" werden vor der Ausformulierung gemischt oder
b) die beiden Komponenten sind jeweils unabhängig voneinander in verschieden Kaugummimassen eingearbeitet, die untereinander lediglich teilverträglich sind, so daß bei der gemeinsamen Kompoundierung beider Grundmassen ein zweiphasiges System entsteht.

2. Kaugummmi-Formulierung gemäß Anspruch 1, mit folgender Zusammensetzung:
| | |
|---|---|
| Acetylsalicylsäure | 2 - 30 Gew.-Teile entsprechend 30 - 1500 mg |
| Basische Puffersubstanzen | entsprechend 0,1-17 mEq Pufferkapazität |
| Kaugummimasse | 15 - 50 Gew.-Teile |
| Weichmacher | 0 - 30 Gew.- Teile |
| Zucker und/oder | 0 - 55 Gew.- Teile |
| Zuckeraustauschstoffe Süßstoff | 0 - 2 Gew.- Teile |
| Aromastoffe | 0 - 5 Gew.- Teile |
| Füllstoffe | 0 - 30 Gew.- Teile |
| ggf. weitere Komponenten wie Wachse, Emulgatoren, Stabilisatoren | 0 - 20 Gew.- Teile |
| ggf. wasserlösliches Polymer | 0 - 30 Gew.- Teile |

3. Kaugummi-Formulierung gemäß Anspruch 1, enthaltend eine Kaugummimasse mit einem Wassergehalt von max. 1 Gew.-%.

4. Verfahren zur Herstellung von Kaugummi-Formulierungen gemäß Ansprüche 1 - 3, dadurch gekennzeichnet, daß man die Acetylsalicylsäure und die basische Puffersubstanz jeweils getrennt mit den Hilfs- und Zuschlagstoffen in gleiche oder teilverträgliche Kaugummibasen eingearbeitet und die beiden erhaltenen Kaugummimassen nach üblichen Methoden bei Temperaturen zwischen 20 und 85° C in eine geeignete Applikationsform überführt.

## Patentansprüche (Patentansprüche für folgende(n) Vertragsstaat(en): ES, GR)

1. Verfahren zur Herstellung von stabiler, schleimhautverträglicher Kaugummiformulierung, die ASS (Acetylsalicylsäure) als eine Komponente und eine zur Salzbildung geeignete basische Substanz als zweite Komponente in räumlich getrennter Form enthält, wobei die beiden Komponenten während des Kauvorganges aus der Kaugummimasse herauslösbar sind und gutlösliche Salze der ASS bilden und die Kaugummimasse maximal 2 Gew.-% Wasser enthält, dadurch gekennzeichnet, daß man die ASS und die basische Puffersubstanz jeweils getrennt mit den Hilfs- und Zuschlagstoffen in gleiche oder teilverträgliche Kaugummibasen eingearbeitet und die beiden erhaltenen Kaugummimassen nach üblichen Methoden bei Temperaturen zwischen 20 und 85°C in eine geeignete Applikationsform überführt.

## Claims (Claims for the following Contracting State(s): AT, BE, CH, DE, DK, FR, GB, IT, LI, LU, NL, SE)

1. Stable chewing gum formulation which is tolerated by the mucous membrane and contains acetylsalicylic acid as one component and a basic substance suitable for salt formation as the second component in a spatially separated form, the two components being able to be dissolved out of the chewing gum composition during the chewing operation and forming readily soluble salts of acetylsalicylic acid and the chewing gum composition containing not more than 2% by weight of water, characterised in that the spatial separation between ASA and the basic buffer substance is ensured by one of the following measures:
a) the two components are in each case incorporated independently of one another in an adequate portion of the chewing gum composition and these "prebatches" are mixed before shaping, or
b) the two components are in each case incorporated independently of one another in different chewing gum compositions which are only partly compatible with one another, with the result that a two-phase system is formed during joint compounding of the two matrices.

2. Chewing gum formulation according to Claim 1, having the following composition:
| | |
|---|---|
| Acetylsalicylic acid | 2 - 30 parts by weight |
| | corresponding to 30-1500 mg |
| Basic buffer substances | corresponding to 0.1-17 meq buffer capacity |
| Chewing gum composition | 15 - 50 parts by weight |
| Plasticiser | 0 - 30 parts by weight |
| Sugar and/or sugar substitutes | 0 - 55 parts by weight |
| Sweetener | 0 - 2 parts by weight |
| Aroma substances | 0 - 5 parts by weight |
| Fillers | 0 - 30 parts by weight |
| if appropriate other components such as waxes, emulsifiers, stabilisers | 0 - 20 parts by weight |
| if appropriate water-soluble polymer | 0 - 30 parts by weight. |

3. Chewing gum formulation according to Claim 1, containing a chewing gum composition having a water content of not more than 1% by weight.

4. Process for the preparation of chewing gum formulations according to Claims 1 - 3, characterised in that the acetylsalicylic acid and the basic buffer substance are in each case incorporated separately with the auxiliaries and additives into chewing gum bases which are identical or partly compatible, and the resultant two chewing gum compositions are converted by customary methods into a suitable application form, at temperatures between 20 and 85°C.

## Claims (Claims for the following Contracting State(s): ES, GR)

1. Process for the preparation of stable chewing gum formulation which is tolerated by the mucous membrane and contains ASA (acetylsalicylic acid) as one component and a basic substance suitable for salt formation as the second component in a spatially separated form, the two components being able to be dissolved out of the chewing gum composition during the chewing operation and forming readily soluble salts of ASA and the chewing gum composition containing not more than 2 % by weight of water, characterised in that the ASA and the basic buffer substance are in each case incorporated separately with the auxiliaries and additives into chewing gum bases which are identical or partly compatible, and the resultant two chewing gum compositions are converted by customary methods into a suitable application form, at temperatures between 20 and 85°C.

## Revendications (Revendications pour l'(les) Etat(s) contractant(s) suivant(s): AT, BE, CH, DE, DK, FR, GB, IT, LI, LU, NL, SE)

1. Formulation de gomme à mâcher stable, n'agressant pas les muqueuses, contenant, sous une forme séparée dans l'espace, de l'acide acétylsalicylique comme premier composant et une substance basique appropriée à la formation de sel comme deuxième composant, les deux composants pouvant être éliminés de la matière de la gomme à mâcher au cours de la mastication et formant des sels bien solubles de l'acide acétylsalicylique et la masse de gomme à mâcher contenant au maximum 2 % en poids d'eau, caractérisée en ce que la séparation dans l'espace de l'AAS et de la substance tampon basique est garantie par les mesures suivantes:
a) chacun des deux composants est incorporé, indépendamment de l'autre, dans une partie adéquate de la matière de la gomme à mâcher et ces "pré-lots" sont mélangés avant la formation, ou
b) chacun des deux composants est incorporé, indépendamment de l'autre, dans des matières de gomme à mâcher, qui sont seulement en partie compatibles entre elles, de sorte qu'un système à deux phases résulte du compoundage commun des deux masses principales.

2. Formulation de gomme à mâcher selon la revendication 1 dont la composition est la suivante:
| | |
|---|---|
| Acide acétylsalicylique | 2-30 parties en poids de manière à obtenir 30-1500 mg |
| Substances tampon basiques | de manière à obtenir 0,1-17 mEq de pouvoir tampon |
| Base de gomme à mâcher | 15 à 50 parties en poids |
| Agent plastifiant | 0 à 30 parties en poids |
| Sucre et/ou édulcorants | 0 à 55 parties en poids |
| Saccharine | 0 à 2 parties en poids |
| Arômes | 0 à 5 parties en poids |
| Matières de charge | 0 à 30 parties en poids |
| éventuellement d'autres | 0 à 20 parties en poids |
| composants (cire, émulsifiants, stabilisateurs) éventuellement le polymère hydrosoluble | 0 à 30 parties en poids |

3. Formulation de gomme à mâcher selon la revendication 1, contenant une masse de gomme à mâcher possédant une teneur en eau de maximum 1 % en poids.

4. Procédé pour préparer des formulations de gomme à mâcher selon les revendications 1 à 3, caractérisé en ce que l'acide acétylsalicylique et la substance tampon basique sont incorporés séparément dans des bases de gomme identiques ou partiellement compatibles avec des adjuvants et des additifs, et les deux masses de gomme à mâcher obtenues sont transformées en une forme d'administration appropriée selon des procédés usuels à des températures comprises entre 20 et 85 °C.

## Revendications (Revendications pour l'(les) Etat(s) contractant(s) suivant(s): ES, GR)

1. Procédé de préparation de gomme à mâcher stable, n'agressant pas les muqueuses, contenant de l'AAS (acide acétylsalicylique) comme premier composant et une substance basique appropriée à la formation de sel comme deuxième composant sous forme séparée dans l'espace, les deux composants pouvant être éliminés de la matière de la gomme à mâcher au cours de la mastication et formant des sels bien solubles de l'acide acétylsalicylique et la masse de gomme à mâcher contenant au maximum 2 % en poids d'eau, caractérisé en ce que l'acide acétylsalicylique et la substance tampon basique sont incorporés dans des bases de gomme identiques ou partiellement compatibles avec des adjuvants et des additifs, et les deux masses de gomme à mâcher obtenues sont transformées en une forme d'administration appropriée selon des procédés usuels, à des températures comprises entre 20 et 85 °C.
